# EUROPEAN PATENT APPLICATION

(11) **EP 1 215 286 A2**
(43) Date of publication of application: **19.06.2002**
(21) Application number: 01129615.9
(22) Date of filing: 12.12.2001
(51) Int. Cl.: C12Q 1/68

(54) **Detection method and detection kit for PCR-amplified base sequences**

(30) Priority: 14.12.2000 JP 2000380465
(71) Applicant: Hitachi Software Engineering Co., Ltd., Yokohama-shi, Kanagawa 231-8475 (JP)
(72) Inventor: Nakao, Motonao, Hitachi Software Engineer. Co. Ltd, Naka-ku, Yokohama-shi, Kanagawa 231-8475 (JP); Mizuno, Katsuya, Hitachi Software Enginee. Co. Ltd, Naka-ku, Yokohama-shi, Kanagawa 231-8475 (JP); Yoshii, Junji, Hitachi Software Engineer. Co., Ltd, Naka-ku, Yokohama-shi, Kanagawa 231-8475 (JP); Asai, Akihiro, Hitachi Software Engineer. Co., Ltd, Naka-ku, Yokohama-shi, Kanagawa 231-8475 (JP)
(74) Representative: Liesegang, Roland, Dr.-Ing.

(57) **Abstract**

Determination of PCR products amplified by PCR can be readily and surely conducted even if multiple types of primers are used therein. By using a biochip of a glass slide with primers for the PCR implanted thereon, competitive PCR is performed in one tube with the multiple types of primers, and amplified PCR products are detected by use of the primers on the biochip.

## Description

### BACKGROUND OF THE INVENTION

### Field of the Invention

The present invention relates to a method of detecting base sequences amplified by a PCR method, and to a detection kit used for the method of detecting base sequences.

### Prior Art

A polymerase chain reaction (PCR) is a method of amplifying specified DNA by use of two types of specific primers. Detection of the DNA amplified by the PCR is generally conducted in a manner that the DNA is subjected to electrophoresis using agarose gel, acrylamide gel or the like, and then it is subjected to dyeing with nucleic acid-specific dyeing reagents. In the case of detecting double-stranded DNA, usually a fluorescent reagent such as ethidium bromide is allowed to enter between two strands of the DNA and then the fluorescent reagent is excited by an ultraviolet light source. As ethidium bromide entered between the two strands of the DNA emits fluorescence, detection is performed by means of capturing the fluorescence with a CCD camera or the like.

However, separation of DNA by the electrophoresis using the agarose gel or the like normally stands on the base number of the DNA. In other words, the electrophoresis can hardly separate a plurality of DNA having the same base number thereof. Therefore, in the case of performing the PCR in a state where a plurality of primers are mixed for the purpose of amplification of DNA being a mold, it is impossible to perform detection by means of separation by the electrophoresis in a case if DNA fragments of potential amplification mutually possess the same base number therein.

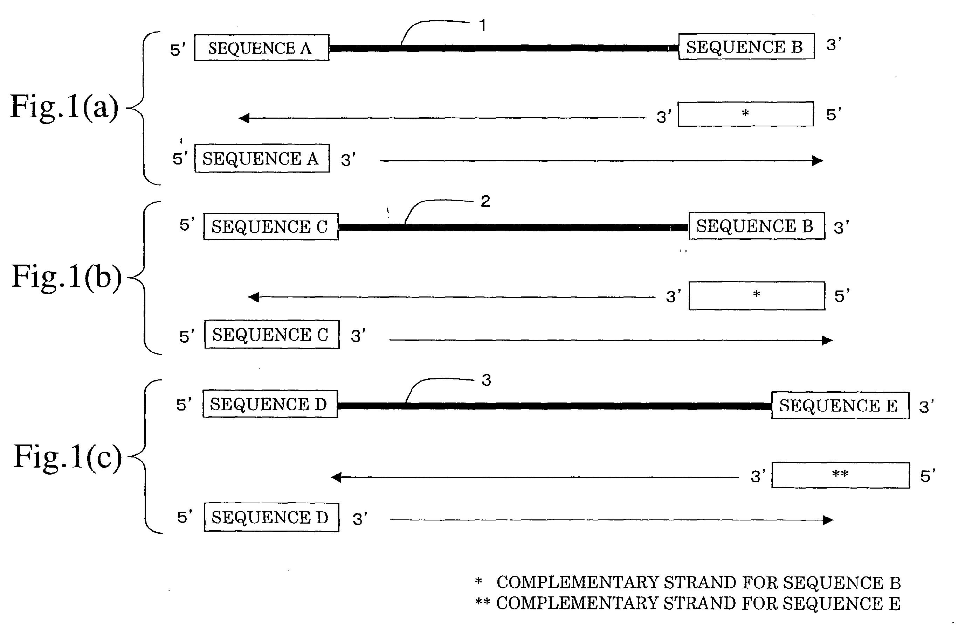

For example, assuming that DNA (1) starting from a sequence A to a sequence B, shown in Fig. 1(a), and DNA (2) starting from a sequence C to the sequence B, shown in Fig. 1(b), are both present, and in the case where amplification of the both DNA (1) and DNA (2) is conducted simultaneously, then the PCR is performed by using three types of primers, each of which includes the sequence A, a complementary strand for the sequence B, or the sequence C, respectively. This mode can be applied to detection of a mutation of a living organism (where mutation is likely to occur at the sequence A or the sequence C; and the sequence B resides in a conserved region), detection of a gene of a virus, or the like.

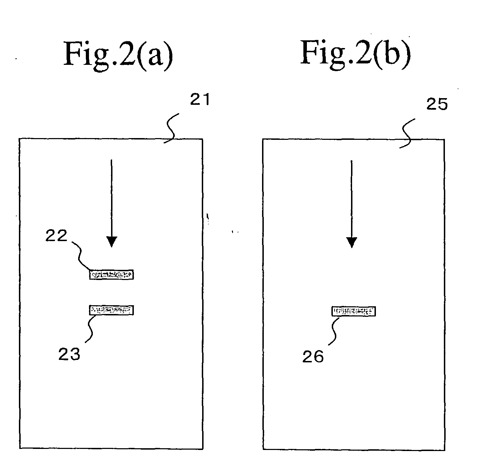

Figs. 2(a) and 2(b) are schematic diagrams showing results of detection by the electrophoresis after conducting the PCR by use of the three kinds of primers simultaneously. Arrows in the drawings indicate directions of the electrophoresis. In the case where the base numbers of the DNA (1) and the DNA (2) are different, as shown in Fig. 2(a), for example, the DNA (1) emerges as a band 22 on gel 21 and the DNA (2) emerges as a band 23, as a result of the electrophoresis. Accordingly, detection of these two types of DNA is feasible by separation. On the contrary, in the case where the DNA (1) and the DNA (2) have the same base number, as shown in Fig. 2(b), only one band 26 emerges on gel 25 upon the electrophoresis. Here, it is indiscernible as whether the DNA on the band 26 is either the DNA (1) or the DNA (2) specifically amplified by any one of the primers, or a mixture of the DNA (1) and the DNA (2) amplified by the both primers. For example, in the case where the DNA (2) is a mutant of the DNA (1) wherein its sequence A is replaced by the sequence C, since the DNA (1) and the DNA (2) have the same base number, it is extremely difficult to conduct separation or detection of DNA (1) and DNA (2) by the electrophoresis .

In such a case, it is necessary to adopt a mode of conducting the PCR individually regarding the DNA (1) and the DNA (2) by means of: preparing two tubes for the PCR; feeding the sequence A and the complementary strand for the sequence B, as primers for PCR amplification of the DNA (1), into one of the tubes; and feeding the sequence C and the complementary strand for the sequence B, as primers for the PCR amplification of the DNA (2), into the other tube. Alternatively, lengths of the DNA fragments of potential amplification need to be modified. For example, if the PCR for a portion of DNA (3) starting from a sequence D to a sequence E, shown in Fig. 1(c), is conducted instead of the PCR for a portion of the DNA (2) shown in Fig. 1(b), a result of the electrophoresis thereof will be similar to the one shown in Fig. 2(a), because of the difference in the base number between the DNA (1) and the DNA (3). Accordingly, independent detection of the amplified fragments of the DNA becomes feasible.

As described above, according to conventional methods, determination of the primers contributed to the amplification of the DNA has been impossible in the case of the PCR conducted inside one tube by use of multiple primers, when amplified products by the multiple primers have the same length. Moreover, in the case of conducting the PCR so as to enable determination by electrophoresis, it has been necessary to modify base numbers among the DNA fragments to be amplified. Such a modification in the base numbers may result in variation of rates of amplification among the respective DNA fragments; therefore, it may complicate comparison of DNA amounts.

### SUMMARY OF THE INVENTION

In consideration of the foregoing problems of the conventional technologies, the present invention purports an object to provide a method to effectuate easy and reliable determination of amplified products by PCR, even in the case where a plurality of base sequences are amplified by the PCR using multiple primers in one tube, and to provide a detection kit for use in such a method.

The present invention achieves the foregoing object by a series of steps of: amplifying a plurality of base sequences in a sample simultaneously by the PCR while using multiple primers; conducting hybridization of the amplified base sequences on a glass slide (a biochip) on which the foregoing PCR primers are implanted; and individually detecting the amplified base sequences by use of the primers. By using the biochip with the primers for the PCR fixed thereon, a base sequence PCR-amplified by its corresponding primer is hybridized in a portion where the corresponding primer is implanted, whereby detection thereof becomes feasible.

Specifically, a method of detecting PCR-amplified base sequences according to the present invention comprises the steps of: conducting PCR amplification by mixing a plurality of pairs of primers with a sample, the primers being suitable for amplifying different base sequences by PCR individually; conducting a hybridization reaction by using a substrate on which the primers used for the PCR are spotted and a solution containing base sequences that are PCR-amplified in the preceding step, the hybridization reaction being performed between the primers spotted on the substrate and the PCR-amplified base sequences; and detecting spots on the substrate in which hybridization occurs.

Detection of PCR products utilizes behavior that a PCR product forms double strands, whereby detection becomes feasible when double-strand specific fluorescent reagents or the like are used. According to this method, those not amplified by the PCR do not form double strands; therefore, they are not detected. Accordingly, only those amplified by the PCR become specifically detectible. In other words, the step of detecting spots on the substrate in which hybridization occurs can be defined as including the steps of: processing a fluorescent material such as ethidium bromide to enter in double-stranded DNA; and detecting fluorescence generated by exciting the fluorescent material contained in any of the spots on the substrate.

It is preferable that each of the primers to be used for the PCR amplification and to be fixed onto the substrate has a base number in a range from 10 to 30. If the base number of the primer exceeds this range, detection by hybridization becomes difficult. It is more preferable to set the range of the base number of the primer from 20 to 25. When the primer has the base number in this range, the PCR-amplified base sequences can be surely detected.

A detection kit according to the present invention is a detection kit for detecting PCR-amplified base sequences, which comprises: a container containing a mixture of at least three types of primers, including two types of primers suitable for amplifying a first base sequence specifically by PCR and two types of primers suitable for amplifying a second base sequence different from the first base sequence specifically by PCR; and a substrate on which a plurality of primers selected from the primers mixed in the container are spotted.

In the event of detecting the PCR-amplified base sequences by use of the detection kit, a sample is first fed in the container for PCR amplification, and then the container after the PCR amplification is subjected to a hybridization reaction with the PCR primers spotted on the substrate. Thereafter, the PCR-amplified base sequences are detected. As for the primers to be used for the detection kit, it is preferable that each of the primers has a base number in a range from 10 to 30. If the base number of the primer exceeds this range, detection by hybridization becomes difficult. It is more preferable to set the range of the base number of the primer from 20 to 25. When the primer has the base number in this range, the PCR-amplified base sequences can be surely detected.

### BRIEF DESCRIPTION OF THE DRAWINGS

Figs. 1(a) to 1(c) are explanatory views concerning primers in a case of conducting PCR in which DNA is used as a mold.
Figs. 2(a) and 2(b) are views describing a method of detecting PCR products by electrophoresis.
Fig. 3 is a schematic plan view of a primer biochip.
Fig. 4 is a view describing a method of detecting PCR products by use of the primer biochip.
Fig. 5 is a view showing a state of fluorescence detection of the primer biochip.

### DETAILED DESCRIPTION OF THE PREFERRED EMBODIMENT

Hereinbelow, an embodiment of the present invention will be described with the accompanying drawings.

An experiment was conducted using a genomic RNA of a hepatitis C virus (HCV) as a sample. The HCV is a retrovirus having RNA in its genomes. Being an RNA-type retrovirus, the HCV is very susceptible to mutations. In fact, a variety of its subtypes have been recognized. As for a method of identifying the subtypes of the HCV, currently adopted is a method of designing specific primers oriented to specific sequences on the subtypes for use in PCR, whereby the subtypes of the HCV become identifiable based on detection results. For example, in Figs 1(a) to 1(c), the sequence A and the sequence C of the respective DNA (1) and DNA (2) are regarded as subtype-specific primers, respectively. Meanwhile, the sequence B is regarded as a common complementary strand for the HCV.

Partial descriptions for three types of genomic sequences of the HCV virus are quoted as follows:

HCV name: AF054256
TGCTCCGCTATGTACGTGGGGGATCTCTGCGGATCTATTTCCTTGTCTC
CCAGCTGTTCACCTTCTCGCCTCGCCGGCATGAGACAGTGCAGGACTG
CAACTGCTCAATCTACCCCGGCCATGTATCAGGTCACCGCATGGCTTGG
GATATGATGATGAACTGGTCACCTACAACAGCCCTAGTGGTGTCGCAGT
TGCTCCGGATCCCACAAGCTGTCGTGGACATGGTGGCGGGGGCCCACT

GGGGAGTCCTGGCGGGCCTTGCCTACTATTCCATGGTAGGGAACTGGG
CTAAGGTTCTGATTGTGGCGCTACTCTTTGCCGGCGTTGACGGG
(Sequence #1)

HCV name: AB008441
TGCTCCGCCATGTACGTGGGAGATCTCTGCGGATCTGTTTTCCTCATCT
CCCAGCTGTTCACCTTCTCACCTCGCCAGTATGAGACGGTACAAGACTG
CAATTGCTCACTCTATCCCGGCCACGTATCAGGTCACCGCATGGCTTGG
GATATGATGATGAACTGGTCACCTACAACAGCCCTGGTGGTATCGCAGT
TGCTCCGGATCCCACAAGCCGTCGTGGACATGGTGGCGGGGGCCCACT
GGGGAGTCCTAGCGGGCCTTGCCTACTATTCCATGGTGGGGAACTGGG
CTAAGGTTCTGATTGTGATGCTACTCTTTGCCGGCGTCGACGGG
(Sequence #2)

HCV name: AF011753
TGCTCGGCCCTCTACGTGGGGGACCTGTGCGGGTCTGTCTTTCTTGTTG
GTCAACTGTTTACCTTCTCTCCCAGGCGCCACTGGACGACGCAAGACTG
CAATTGTTCTATCTATCCCGGCCATATAACGGGTCATCGCATGGCATGGG
ATATGATGATGAACTGGTCCCCTACGGCAGCGTTGGTGGTAGCTCAGCT
GCTCCGGATCCCACAAGCCATCATGGACATGATCGCTGGTGCTCACTGG
GGAGTCCTGGCGGGCATAGCGTATTTCTCCATGGTGGGGAACTGGGCG
AAGGTCCTGGTAGTGCTGCTGCTATTTGCCGGCGTCGACGCG
(Sequence #3)

In order to identify the respective names of these HCV genomes, a sequential portion common to all of the genome sequences, and sequential portions specific in the respective genome sequences are utilized. Since a sequential portion of positions from 194 to 216 has a sequence TGCTCCGGATCCCACAAGC (Sequence #4) (comprising 19 bases) that is common to all of the genomes, such a portion is used as a common portion. On the contrary, the HCV genomes have different base sequences from one another in a portion corresponding to positions from 41 to 60, namely, TCCTTGTCTCCCAGCTGTTC (Sequence #5) in AF054256, TCCTCATCTCCCAGCTGTTC (Sequence #6) in AB008441 and TTCTTGTTGGTCAACTGTTT (Sequence #7) in AF011753. Accordingly, as for a primer for the PCR, a complementary strand sequence corresponding to the common portion (positions from 194 to 216) is used as a common reverse primer (an R-primer). As for the positions from 41 to 60, three types of front primers (F-primers) are severally prepared. The PCR is thereby conducted.

In the case of conducting the PCR on the HCV genomes, it is necessary to reverse transcribe RNA into DNA to begin with. A random primer was used for the reverse transcription, and Superscript 2 (made by GibcoBRL) was used as reverse transcriptase. Using cDNA thus produced as a mold, the PCR was carried out under the following conditions:

| | |
|---|---|
| F-primer (10 pmol/µl) | 1 µl |
| R-primer (10 pmσl/µl) | 1 µl |
| dNTP MIX (2mM each dNTP) | 2.0 µl |
| 10+PCR Buffer | 2.5 µl |
| Taq | 0.1 µl (0.5 U) |
| DDW | 17.4 µl |
| product (cDNA) | 1 µl |
| | |
| Total | 25 µl |

Thermal Cycler setting (about 35 cycles, each cycle composed of the following [1], [2] and [3])
[1] 94.0°C, 1 min.
[2] 60.0°C, 1 min.
[3] 72.0°C, 1 min.

Since PCR products having the same length cannot be detected by separation in a conventional method, it has been impossible to mix F-primers. Accordingly, it has been necessary to perform detection by conducting the PCR individually for the respective F-primers instead of mixing them. On the contrary, the present invention can achieve identification of amplified PCR products even when the PCR is conducted by use of a mixture of the three types of F-primers and the common R-primer.

A mixture of all the primers cited above was added into a tube with a sample therein in advance, and then the PCR was conducted. The PCR products were retrieved by ethanol precipitation, and then dissolved into 10 µl of 4xSSC, 0.2% SDS solution.

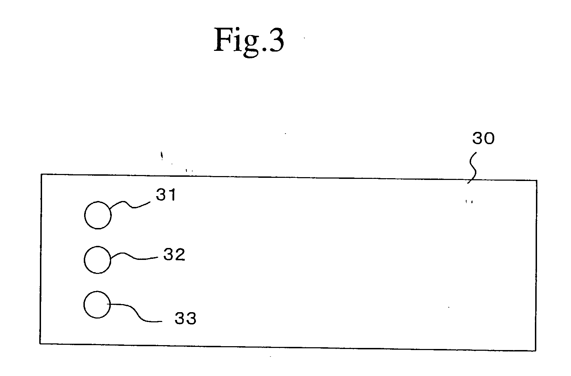

Fig. 3 is a schematic plan view of a biochip used is the experiment. The biochip used therein was fabricated by cross-linking glutaraldehyde with a glass slide 30 coated with aminopropylethoxysilane to expose an aldehyde base at a terminal. Moreover, primers 31, 32 and 33 for the PCR, the primers being implanted on the glass slide 30, were subjected to C6 amination on their terminals in order to enhance fixation rates thereof to the glass slide 30. After spotting the primers 31, 32 and 33 for the PCR on the glass slide 30, sodium borate was dissolved in a phosphate buffer, whereby the spotted primers 31, 32 and 33 were fixed to the glass slide 30. The sequences of the primers used therein are, TCCTTGTCTCCCAGCTGTTC (Sequence #5) (AF054256) for the primer 31, TCCTCATCTCCCAGCTGTTC (Sequence #6) (AB008441) for the primer 32, and TTCTTGTTGGTCAACTGTTT (Sequence #7) (AF011753) for the primer 33, respectively. It should be noted that a method of fixing the primers onto the glass slide 30 is not limited to the foregoing method.

Subsequently, the sample subjected to the PCR as described above was added onto the biochip 30, and then a glass cover was slowly overlaid thereon. The glass slide was then subjected to hybridization for three hours in an incubator maintained at 40°C. Thereafter, the hybridized glass slide was soaked in 1xSSC, 0.2% SDS solution until the glass cover was spontaneously peeled off. Then, the glass slide was soaked in 0.1xSSC solution with addition of a reagent specifically reactive with double-stranded DNA, such as ethidium bromide, cyber green, Hoechst 33258 or the like, for the purpose of dyeing thereof. These reagents possess structures that allow the reagents to specifically enter between double helix structures of the double-stranded DNA, thus effectuating detection of the double-stranded DNA. The reagents to be used for the detection are not limited to those cited above.

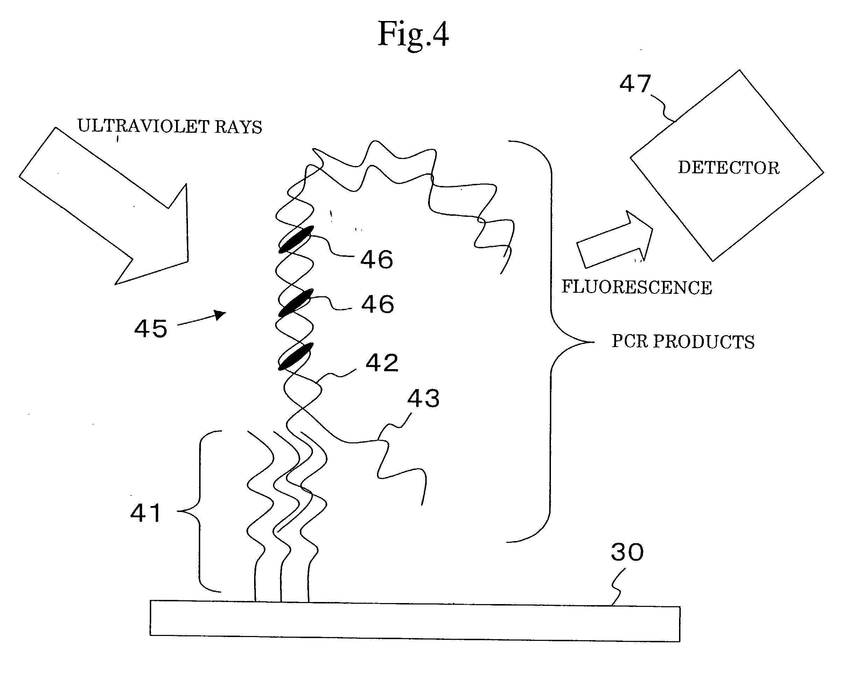

Fig. 4 is a view describing a method of detecting PCR products according to the present invention. PCR-amplified DNA 42 is subjected to hybridization with a portion 41 of primers fixed onto the glass slide 30. The DNA 42 hybridized with the portion 41 is a PCR product; therefore, a complementary strand thereto is also amplified simultaneously. Accordingly, such a complementary strand 43 is further hybridized with the DNA 42 that is hybridized with any one of the primer on the glass slide, whereby double strands of the DNA are formed. A double-strand specific fluorescent reagent 46 enters in a portion 45 of the double-stranded DNA. Therefore, if the PCR product is present in the sample, the double-stranded DNA is present at a spotted position of the corresponding primer on the glass slide 30; likewise, the double-strand specific fluorescent reagent is also present there. DNA not amplified by the PCR is not hybridized with the corresponding primer on the glass slide 30; accordingly, the double-strand specific fluorescent reagent is not present at the spotted position of the corresponding primer. In other words, it becomes possible to identify the type of the PCR-amplified DNA by finding the spot of the primer generating fluorescence in the event of irradiating fluorescence excitation light onto the glass slide.

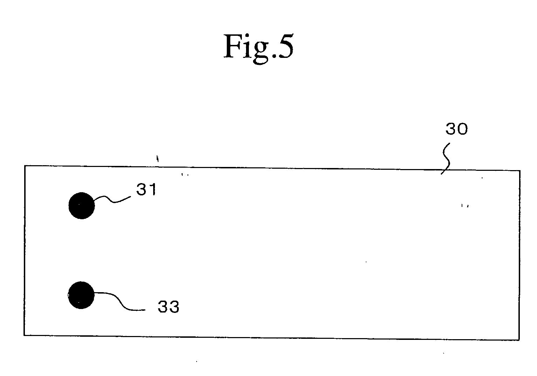

After dyeing the glass slide with the double-strand specific fluorescent reagent 46, the glass slide 30 was dried by a centrifuge. Thereafter, the fluorescent reagent 46 was excited by a transilluminator or the like as shown in Fig. 4, and then fluorescence was captured with a high-sensitive imaging system 47 such as a chilled CCD camera. A result of imaging is illustrated in Fig. 5. From this result, occurrence of hybridization was determined with respect to the primer 31 and the primer 33. In other words, presence of AF054256 and AF011753 was determined in the mold DNA.

According to the present invention, individual rates of amplification on a biochip can be determined even if PCR is conducted within one tube using multiple primers. Moreover, since a sample can be amplified by the PCR, a system with higher sensitivity can be provided compared with a conventional method using a biochip.

## Claims

1. A method of detecting PCR-amplified base sequences, comprising the steps of:
conducting PCR amplification by mixing a plurality of pairs of primers with a sample, said primers being suitable for amplifying different base sequences by PCR individually;
conducting a hybridization reaction by using a substrate on which said primers used for the PCR are spotted and a solution containing said base sequences that are PCR-amplified in the preceding step, said hybridization reaction being performed between the primers spotted on the substrate and said PCR-amplified base sequences; and
detecting spots on said substrate in which hybridization occurs.

2. The method of detecting PCR-amplified base sequences according to claim 1, wherein said step of detecting spots on said substrate in which hybridization occurs includes the steps of:
processing a fluorescent material to enter in double-stranded DNA; and
detecting fluorescence generated by exciting said fluorescent material contained in any of the spots on the substrate.

3. The method of detecting PCR-amplified base sequences according to any one of claims 1 and 2, wherein each of said primers has a base number in a range from 10 to 30.

4. A detection kit for detecting PCR-amplified base sequences, comprising:
a container containing a mixture of at least three types of primers, including: two types of primers suitable for amplifying a first base sequence specifically by PCR; and two types of primers suitable for amplifying a second base sequence different from said first base sequence specifically by the PCR; and
a substrate on which a plurality of primers selected from said primers mixed in the container are spotted.

5. The detection kit according to claim 4, wherein each of said primers has a base number in a range from 10 to 30.


SEQUENCE LISTING

<110> HITACHI SOFTWARE ENGINEERING CO., LTD.

<120> DETECTION METHOD AND DETECTION KIT FOR PCR AMPLIFIED BASE SEQUENCES

<130> PH-1431

<140>
<141>

<160> 7

<170> PatentIn Ver. 2.0

<210> 1
<211> 336
<212> DNA
<213> Hepatitis C virus

<400> 1
tgctccgcta tgtacgtggg ggatctctgc ggatctattt tccttgtctc ccagctgttc 60
accttctcgc ctcgccggca tgagacagtg caggactgca actgctcaat ctaccccggc 120
catgtatcag gtcaccgcat ggcttgggat atgatgatga actggtcacc tacaacagcc 180
ctagtggtgt cgcagttgct ccggatccca caagctgtcg tggacatggt ggcgggggcc 240
cactggggag tcctggcggg ccttgcctac tattccatgg tagggaactg ggctaaggtt 300
ctgattgtgg cgctactctt tgccggcgtt gacggg 336

<210> 2

<211> 336
<212> DNA
<213> Hepatitis C virus

<400> 2
tgctccgcca tgtacgtggg agatctctgc ggatctgttt tcctcatctc ccagctgttc 60
accttctcac ctcgccagta tgagacggta caagactgca attgctcact ctatcccggc 120
cacgtatcag gtcaccgcat ggcttgggat atgatgatga actggtcacc tacaacagcc 180
ctggtggtat cgcagttgct ccggatccca caagccgtcg tggacatggt ggcgggggcc 240
cactggggag tcctagcggg ccttgcctac tattccatgg tggggaactg ggctaaggtt 300
ctgattgtga tgctactctt tgccggcgtc gacggg 336

<210> 3
<211> 336
<212> DNA
<213> Hepatitis C virus

<400> 3
tgctcggccc tctacgtggg ggacctgtgc gggtctgtct ttcttgttgg tcaactgttt 60
accttctctc ccaggcgcca ctggacgacg caagactgca attgttctat ctatcccggc 120
catataacgg gtcatcgcat ggcatgggat atgatgatga actggtcccc tacggcagcg 180
ttggtggtag ctcagctgct ccggatccca caagccatca tggacatgat cgctggtgct 240
cactggggag tcctggcggg catagcgtat ttctccatgg tggggaactg ggcgaaggtc 300
ctggtagtgc tgctgctatt tgccggcgtc gacgcg 336

<210> 4
<211> 19
<212> DNA
<213> Hepatitis C virus

<400> 4
tgctccggat cccacaagc                                                    19

<210> 5
<211> 20
<212> DNA
<213> Hepatitis C virus

<400> 5
tccttgtctc ccagctgttc                                                   20

<210> 6
<211> 20
<212> DNA
<213> Hepatitis C virus

<400> 6
tcctcatctc ccagctgttc                                                   20

<210> 7
<211> 20
<212> DNA
<213> Hepatitis C virus

<400> 7
ttcttgttgg tcaactgttt                                                   20